# EUROPÄISCHE PATENTSCHRIFT

(11) **EP 2 556 817 B1**
(45) Veröffentlichungstag und Bekanntmachung des Hinweises auf die Patenterteilung: **02.01.2019**
(21) Anmeldenummer: 12175509.4
(22) Anmeldetag: 09.07.2012
(51) Int. Cl.: A61K 8/24, A61K 8/25, A61K 8/55, A61Q 11/00

(54) **Mund- und Zahnpflege- und -reinigungsmittel für sensitive Zähne**
Oral and tooth care and cleaning composition for sensitive teeth
Produit de nettoyage et d'entretien de la bouche et des dents pour dents sensibles

(30) Priorität: 12.08.2011 DE 102011080893
(43) Veröffentlichungstag der Anmeldung: 13.02.2013
(73) Patentinhaber: Henkel AG & Co. KGaA, 40589 Düsseldorf (DE)
(72) Erfinder: Adomat, Christel, 40591 Düsseldorf (DE); Duschek, Nicole, 40595 Düsseldorf (DE); Miehlich, Kristin, 42119 Wuppertal (DE)

(56) Entgegenhaltungen:
- EP-A1- 0 785 169
- EP-A2- 0 422 803
- WO-A1-2005/065634
- DE-A1- 10 340 542

## Beschreibung

Die Erfindung betrifft Mund- und Zahnpflegemittel mit einer Wirkstoffkombination zur schonenden und effektiven Reinigung der Zähne.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Viele Menschen wünschen sich weiße Zähne und empfinden dunkle oder verfärbte Zähne als kosmetisch inakzeptabel. Die Aufrechterhaltung der natürlichen Zahnfarbe ist jedoch trotz regelmäßiger Dentalhygiene nicht immer erfolgreich. Ernährungsgewohnheiten oder Rauchen können zu Zahnverfärbungen führen. Ebenso führt die Besiedlung der Zahnoberfläche mit Bakterien (Plaque) zu Verfärbungen.
Eine Reihe von technischen Lösungen zur Entfernung oder Aufhellung von Zähnen wurde entwickelt. Zur Aufhellung/Bleichen kommt vor allem Peroxid zum Einsatz. In professionellen Bleichprodukten wird Peroxid in hohen Konzentrationen eingesetzt, während der Einsatz in Kosmetikprodukten zur Mund- und Zahnpflege auf 0,1% Peroxid beschränkt ist. Peroxid in dieser Konzentration hat nur eine eingeschränkte aufhellende Wirkung und kann Verfärbungen der Zähne oft nicht im gewünschten Maß beseitigen.
Eine weitere Möglichkeit zur Aufhellung der Zähne besteht in der effektiven Entfernung von Zahnbelägen, welche die Zähne dunkler aussehen lassen. Diese Methode der Zahnaufhellung wird auch als "Natural Whitening" beschrieben. Eine hohe Reinigungsleistung wird am besten erreicht durch Putzkörper, zum Beispiel Silica, Alumina oder Calciumcarbonat in Kombination mit einem Tensid. Leider weisen Zahncremes mit einem effektiven System aus einem oder mehreren Putzkörpern häufig auch eine hohe Abrasivität auf, führen also zu einem gewissen, wenn auch sehr geringem Abrieb der Zahnoberfläche. Dies kann sich insbesondere dann nachteilig auswirken, wenn das Enamel eines Zahnes ohnehin dünn ist, wie dies bei Personen mit empfindlichen Zähnen der Fall ist. Oft treten bei Personen mit empfindlichen Zähnen auch freiliegende Zahnhälse auf, also Partien des Zahnes in unmittelbarer Nähe zum Zahnfleisch, an denen kein Enamel als Schutzschicht vorhanden ist und das darunter liegende Dentin frei liegt.

Es besteht daher ein Bedarf an Zahnpasten, die eine effektive Reinigung und Aufhellung bewirken aber dabei gleichzeitig die Zahnoberfläche schützen bzw. remineralisieren. Eine solche Zahnpaste ist besonders sinnvoll für Personen mit empfindlichen Zähnen, die auf eine Whitening-Leistung nicht verzichten wollen.

Die DE 103 40 542 A1 offenbart schonende und remineralisierende Zahncremes, welche in Beispiel 1 5 Gew.-% Sident® 8, 1,1 Gew.-% Natriummonofluorphosphat und 0,52 Gew.-% Nanit® enthalten In der EP 422 803 A2 wird der Einsatz von Sident® 9 in Zahncremes offenbart.

Die WO2005/065634 A1 offenbart den Einsatz von Sorbosil® A33.

In der EP 785 169 A1 wird ein Zusammenhang zwischen Abrasivität und spezifischer Oberfläche von Poliermitteln hergestellt.

Die Aufgabe der vorliegenden Erfindung bestand darin, Zubereitungen zur Mund- und Zahnpflege und -reinigung bereitzustellen, die eine effektive Reinigung und Aufhellung bewirken und dabei trotz guter Reinigungsleistung weniger abrasiv sind als herkömmliche Zahncremes.

Es wurde gefunden dass durch die Kombination eines effektiven Reinigungssystems mit Natriummonofluorophosphat und einem Calciumsalz die schädigende Wirkung gegenüber einer Referenz mit demselben Reinigungssystem und Natriumfluorid verringert werden kann. Insbesondere die Empfindlichkeit der Zähne beim und nach dem Putzvorgang ist im Vergleich zu einer Referenzzahncreme ohne die Kombination aus Natriummonofluorophosphat und Calcium verringert.

Gegenstand der Erfindung sind daher Mund- und Zahnpflege- und -reinigungsmittel, enthaltend - bezogen auf ihr Gewicht -
a) 1 bis 30 Gew.-% Fällungskieselsäure(n) mit einer spezifischen Oberfläche nach ISO 5794-1, Anhang D von ≤ 55 m²/g;
b) 0,1 bis 10 Gew.-% Natriummonofluorphosphat;
c) 0,001 bis 10,0 Gew.-% mindestens eines Calciumsalzes,
wobei
das Gewichtsverhältnis von Fällungskieselsäuren mit einer spezifischen Oberfläche nach ISO 5794-1, Anhang D von ≤ 55 m²/g (Inhaltsstoff a)) zu ggf. vorhandenen Fällungskieselsäuren mit einer spezifischen Oberfläche nach ISO 5794-1, Anhang D von > 55 m²/g > 50:1 beträgt.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes, Mund- und Zahnspülungen sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden. Eine weitere besonders bevorzugte Ausführungsform der vorliegenden Erfindung sind Mundspüllösungen und Mundwasser, die zum Ausspülen der Mundhöhle verwendet werden.

Als ersten wesentlichen Inhaltsstoff enthalten die erfindungsgemäßen Zusammensetzungen bezogen auf ihr Gewicht 1 bis 30 Gew.-% Fällungskieselsäure(n) mit einer spezifischen Oberfläche nach ISO 5794-1, Anhang D von ≤ 55 m²/g. Vorzugsweise werden die Fällungskieselsäuren, die entsprechende spezifische Oberflächen aufweisen, innerhalb engerer Mengenbereiche eingesetzt, und insbesondere bevorzugt werden Fällungskiselsäuren eingesetzt, die noch niedrigere spezifische Oberflächen nach ISO 5794-1, Anhang D aufweisen. Bevorzugte erfindungsgemäße Mund- und Zahnpflege- und - reinigungsmittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 13,0 Gew.-% Fällungskieselsäure(n) mit einer spezifischen Oberfläche nach ISO 5794-1, Anhang D von ≤ 55 m²/g.

Besonders bevorzugte erfinddungsgemäße Mund und Zahnpflege- und -reinigungsmittel sind dadurch gekennzeichnet, dass sämtliche im Mittel enthaltenen Fällungskieselsäure(n) eine spezifische Oberfläche nach ISO 5794-1, Anhang D von ≤ 53 m²/g, vorzugsweise von ≤ 51 m²/g, weiter bevorzugt von ≤ 49 m²/g und insbesondere von ≤ 47 m²/g aufweisen.

In weiter bevorzugten erfindungsgemäßen Mitteln sind die eingesetzten Fällungskieselsäuren durch weitere physikalische Parameter gekennzeichnet. Vorzugsweise einzusetzende Fällungskieselsäuren weisen Stampfdichten > 360 g/l (gemessen nach ISO 787-11), besonders bevorzugt > 375 g/l, weiter bevorzugt > 400 g/l und insbesondere > 425 g/l auf.

Es ist weiter bevorzugt, Fällungskieselsäuren einzusetzen, die eine DBP-Absorption gemäß DIN 53601 von weniger als 140 g/100 g aufweisen. Ganz besonders bevorzugte erfindungsgemäß einzusetzende Fällungskieselsäuren weisen eine DBP-Absorption gemäß DIN 53601 von weniger als 135 g/100 g, bevorzugt von eine DBP-Absorption gemäß DIN 53601 von weniger als 130 g/100 g und insbesondere von weniger als 125 g/100 g auf.

Erfindungsgemäß besonders bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer Stampfdichte (gemessen nach ISO 787-11), von > 425 g/l.

Erfindungsgemäß weiter bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer DBP-Absorption gemäß DIN 53601 von weniger als 125 g/100 g.

Erfindungsgemäß insbesondere bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer Stampfdichte (gemessen nach ISO 787-11), von > 425 g/l und einer DBP-Absorption gemäß DIN 53601 von weniger als 125 g/100 g.

Die erfindungsgemäßen Mittel können zusätzlich zu den genannten Fällungskieselsäuren a) weitere Poliermittel enthalten. Als Poliermittel eignen sich prinzipiell alle für Zahnpasten bekannten Reibkörper, insbesondere solche, die keine Calciumionen enthalten. Bevorzugt geeignete Poliermittel-komponenten sind daher Aluminiumhydroxid, Aluminiumoxid, Natrium-aluminiumsilikate, organische Polymere oder Gemische solcher Reibkörper.

Es ist bevorzugt, dass die erfindungsgemäßen Zusammensetzungen nur wenig bis keine Fällungskieselsäuren enthalten, die eine spezifische Oberfläche nach ISO 5794-1, Anhang D von > 55 m²/g aufweisen. Sollen solche Kieselsäuren eingesetzt werden, liegt das Gewichtsverhältnis von Fällungskieselsäuren mit einer spezifischen Oberfläche nach ISO 5794-1, Anhang D von ≤ 55 m²/g (Inhaltsstoff a)) zu Fällungskieselsäuren mit einer spezifischen Oberfläche nach ISO 5794-1, Anhang D von > 55 m²/g erfindungsgemäß bei > 50:1.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich. Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Als zweiten wesentlichen Inhaltsstoff enthalten die erfindungsgemäßen Mittel 0,1 bis 10 Gew.-% Natriummonofluorphosphat. Gegenüber anderen Antikaries-Wirkstoffen wie beispielsweise organischen oder anorganischen Fluoriden, z. B. Natriumfluorid, Kaliumfluorid und Natriumfluorosilikat, Zinkfluorid oder Zinn-(II)-fluorid hat der Einsatz von Natriummonofluorphosphat in Kombination mit der speziellen Fällungskieselsäure und dem Calciumsalz den Vorteil, dass die schädigende Wirkung verringert werden kann. Insbesondere die Empfindlichkeit der Zähne beim und nach dem Putzvorgang ist deutlich verringert

Erfindungsgemäß bevorzugte Mund und Zahnpflege- und -reinigungsmittel enthalten 0,01 bis 5 Gew.-%, vorzugsweise 0,1 bis 3 Gew.-%, weiter bevorzugt 0,2 bis 2 Gew.-% und insbesondere 0,5 bis 1,2 Gew.-% Natriummonofluorphosphat.

Als dritten wesentlichen Inhaltsstoff enthalten die erfindungsgemäßen Mittel 0,001 bis 10,0 Gew.-% mindestens eines Calciumsalzes. Besonders bevorzugt werden Calciumsalz(e) innerhalb engerer Mengenbereiche eingesetzt, so dass bevorzugte Mund und Zahnpflege- und -reinigungsmittel 0,05 bis 7,5 Gew.-%, vorzugsweise 0,1bis 5 Gew.-%, weiter bevorzugt 0,15 bis 2,5 Gew.-% und insbesondere 0,2 bis 1,25 Gew.-% Calciumsalz(e) enthalten.

Es lassen sich erfindungsgemäß alle physiologisch verträglichen Calciumsalze einsetzen, bevorzugt ist der Einsatz von Calciumsalzen, die einen weiteren Nutzen im erfindungsgemäßen Mund und Zahnpflege- und -reinigungsmittel entfalten. Unter diesen Verbindungen ganz besonders bevorzugt sind Calciumhydrogenphosphat-Dihydratn und/oder Calcium-Glycerophosphat.

Calciumhydrogenphosphat-Dihydrat, CaHPO₄•2H₂O wird je nach Literaturstelle auch als "Bruschit" oder als Dicalciumphosphat-dihydrat bezeichnet. Erfindungsgemäß bevorzugt ist der Einsatz von CaHPO₄•2H₂O, das durch die CAS-Nr: 7789-77-7 beschrieben wird.

Erfindungsgemäße Mund und Zahnpflege- und -reinigungsmittel, die als Calciumsalz Calciumhydrogenphosphat-Dihydrat, CaHPO₄•2H₂O enthalten, weisen deutliche Abrasivitätsvorteile gegenüber anderen Mitteln auf, die schonende Reinigung sensitiver Zähne ist daher mit der erfindungsgemäßen Kombination noch einmal deutlich besser, wenn Calciumhydrogenphosphat-Dihydrat als Calciumsalz eingesetzt wird.

Es hat sich gezeigt, dass Calciumhydrogenphosphat-Dihydrat, CaHPO₄•2H₂O, vorzugsweise innerhalb enger Mengenbereiche eingesetzt wird. Hier sind erfindungsgemäße Mund und Zahnpflege- und -reinigungsmittel bevorzugt, die 0,25 bis 7,5 Gew.-%, vorzugsweise 0,5 bis 7,0 Gew.-%, besonders bevorzugt 1,0 bis 6,0 Gew.-%, weiter bevorzugt 2,0 bis 5,0 Gew.-% und insbesondere 3,5 bis 4,5 Gew.-% Calciumhydrogenphosphat-Dihydrat enthalten.

Zusätzlich zu Calciumhydrogenphosphat-Dihydrat oder an seiner Stelle können die erfindungsgemäßen Mittel mit besonderem Vorzug Calcium-Glycerophosphat, d.h. ein Calciumsalz mindestens einer Glycerophosphorsäure enthalten.

Die Glycerophosphorsäure ist eine zweibasige Säure, die in zwei isomeren Formen vorkommt, je nachdem, ob die Phosphorsäuregruppierung an eine terminale oder die mediale OH-Gruppe des Glycerins gebunden vorliegt. Die Form, bei der die Phosphorsäuregruppierung an eine terminale OH-Gruppe des Glycerins gebunden vorliegt, wird auch als alpha-Isomer, die Form, bei der die Phosphorsäuregruppierung an die mediale OH-Gruppe des Glycerins gebunden vorliegt, auch als beta-Isomer bezeichnet.

Das alpha-Isomer ist zusätzlich optisch aktiv und tritt in den zwei enantiomeren Formen sn-Glycerol-1-phosphorsäure 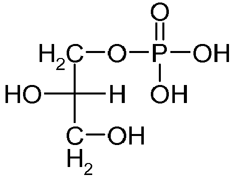 sowie sn-Glycerol-3-phosphorsäure 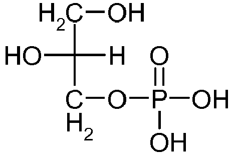 auf.

Das Präfix sn bei Glycerol-Derivaten steht für "stereospezifisch nummeriert" und verlangt, dass die 2-Hydroxy-Gruppe in der vorstehend verwendeten Fischer-Projektion nach links weist. Glycerol-2-phosphat ist nicht optisch aktiv. Die Glycerophosphorsäuren sind etwa so stark wie Phosphorsäure.

Erfindungsgemäß bevorzugt ist der Einsatz des alpha-Isomers, unabhängig davon, welches der beiden Enantiomere eingesetzt wird. Sofern der Einsatz enantiomerenreiner Verbindungen gewünscht ist, wird vorzugsweise das Calciumsalz der sn-Glycerol-3-phosphorsäure eingesetzt.

Zusammenfassend sind erfindungsgemäße Mund und Zahnpflege- und -reinigungsmittel bevorzugt, die Calciumsalze der Glycerophosphorsäuren der Formeln (Ia) und (Ib) enthalten

HO-CH₂-CH(OH)-CH₂-OP(O)O₂²⁻ Ca²⁺ (Ia) HO-CH₂-CH(OP(O)O₂²⁻)-CH₂-OH Ca²⁺ (Ib).

Hierbei sind besonders bevorzugte erfindungsgemäße Mund und Zahnpflege- und reinigungsmittel dadurch gekennzeichnet, dass das Gewichtsverhältnis der Calciumsalze der Formeln (Ia) zu (Ib) oberhalb von 50:50, vorzugsweise oberhalb von 60:40, besonders bevorzugt oberhalb von 70:30 und insbesondere oberhalb 80:20 liegt.

Der Einsatz der Calcium-Glycerophosphate innerhalb engerer Mengenbereiche ist bevorzugt. Bevorzugte erfindungsgemäße Mund- und Zahnpflege- und -reinigungsmittel enthalten - bezogen auf ihr Gewicht - 0,01 bis 2,5 Gew.-%, vorzugsweise 0,05 bis 2,0 Gew.-%, besonders bevorzugt 0,1 bis 1,0 Gew.-%, weiter bevorzugt 0,11 bis 0,75 Gew.-% und insbesondere 0,12 bis 0,5 Gew.-% Calcium-Glycerophosphat.

Ganz besonders bevorzugte erfindungsgemäße Mittel enthalten 0,25 bis 7,5 Gew.-%, vorzugsweise 0,5 bis 7,0 Gew.-%, besonders bevorzugt 1,0 bis 6,0 Gew.-%, weiter bevorzugt 2,0 bis 5,0 Gew.-% und insbesondere 3,5 bis 4,5 Gew.-% Calciumhydrogenphosphat-Dihydrat und 0,01 bis 2,5 Gew.-%, vorzugsweise 0,05 bis 2,0 Gew.-%, besonders bevorzugt 0,1 bis 1,0 Gew.-%, weiter bevorzugt 0,11 bis 0,75 Gew.-% und insbesondere 0,12 bis 0,5 Gew.-% Calcium-Glycerophosphat.

Erfindungsgemäße Mund und Zahnpflege- und -reinigungsmittel können weitere Inhaltsstoffe enthalten. Es hat sich gezeigt, dass bestimmte Magnesiumsalze die synergistische Wirkung der erfindungsgemäßen Kombination weiter steigern können. Demnach sind erfindungsgemäße Mund und Zahnpflege- und -reinigungsmittel besonders bevorzugt, die 0,25 bis 7,5 Gew.-%, vorzugsweise 0,5 bis 6,0 Gew.-%, besonders bevorzugt 1,0 bis 5,0 Gew.-%, weiter bevorzugt 1,5 bis 3,0 Gew.-% und insbesondere 1,75 bis 2,5 Gew.-% Magnesiumsulfat-Heptahydrat enthalten. Der Einsatz von Magnesiumsulfat-Heptahydrat ist besonders bevorzugt, wenn als Ca-Salz Calciumhydrogenphosphat-Dihydrat eingesetzt wird. In besonders bevorzugten erfinddungsgemäßen Mund und Zahnpflege- und -reinigungsmitteln beträgt das Gewichtsverhältnis von Magnesiumsulfat-Heptahydrat zu Calciumhydrogenphosphat-Dihydrat 20:1 bis 1:1, vorzugsweise 15:1 bis 2:1, weiter bevorzugt 10:1 bis 5:2, besonders bevorzugt 4:1 bis 3:1 und insbesondere 18:7 bis 15:7.

Auch oberflächenaktive Substanzen sind in den erfindungsgemäßen Mitteln einsetzbar. Sie dienen beispielsweise in Zahnpasten zur Unterstützung der Reinigungswirkung und gewünschtenfalls auch zur Entwicklung von Schaum beim Zähnebürsten oder beim Mundspülen sowie zur Stabilisierung der Polierkörperdispersion im Träger und werden sowohl in Mundspüllösungen als auch in Zahnpasten üblicherweise in einer Menge von 0,1-5 Gew.-% eingesetzt.
Geeignete Tenside sind z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂- C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆)-estern, Acylsarcosinen, Acyltauriden und Acylisothionaten mit jeweils 8-18 C-Atomen in der Acylgruppe. Auch zwitterionische, ampholytische und nichtionische Tenside sind geeignet, z. B. Oxethylate von Fettsäuremono- und -diglyceriden, von Fettsäure-Sorbitanestern und Alkyl(oligo)-Glucoside sowie Fettsäureamidoalkylbetaine.

Es ist erfindungsgemäß bevorzugt, den Einsatz von Tensiden weitestgehend zu beschränken, um die desensibilisierende Wirkung der erfindungsgemäßen Kombination noch deutlicher in den Vordergrund treten lassen zu können. Daher sind erfindungsgemäße Mund und Zahnpflege- und - reinigungsmittel, die - bezogen auf ihr Gewicht - weniger als 5 Gew.-%, vorzugsweise weniger als 4 Gew.-%, besonders bevorzugt weniger als 3 Gew.-% und insbesondere weniger als 2 Gew.-% Tensid(e) enthalten, erfindungsgemäß besonders bevorzugt.

Ganz besonders bevorzugt ist es, insbesondere den Einsatz anionischer Tenside weitestgehend zu beschränken oder ganz auf diese Tenside zu verzichten. Hier sind bevorzugte erfindungsgemäße Mund und Zahnpflege- und -reinigungsmittel dadurch gekennzeichnet, dass sie weniger als 2 Gew.-%, vorzugsweise weniger als 1 Gew.-%, besonders bevorzugt weniger als 0,5 Gew.-% und insbesondere weniger als 0,1 Gew.-% anionische(s) Tensid(e) enthalten, wobei bevorzugte Mittel frei von anionischen Tensiden sind.

Sofern Tenside - vorzugsweise innerhalb der vorstehend genannten Höchstgrenzen - eingesetzt werden sollen, ist der Einsatz amphoterer Tenside bevorzugt. Bevorzugte tensidhaltige erfindungsgemäße Mund und Zahnpflege- und -reinigungsmittel enthalten 0,1 bis 5 Gew.-%, vorzugsweise 0,25 bis 4 Gew.-%, besonders bevorzugt 0,5 bis 3,0 Gew.-%, weiter bevorzugt 0,75 bis 2,0 Gew.-% und insbesondere 1,0 bis 1,5 Gew.-% amphotere(s) Tensid(e).

Insbesondere bevorzugte erfindungsgemäße Mund und Zahnpflege- und -reinigungsmittel dieser Ausführungsform enthalten 0,1 bis 5 Gew.-%, vorzugsweise 0,2 bis 4 Gew.-%, besonders bevorzugt 0,25 bis 3 Gew.-%, weiter bevorzugt 0,3 bis 2 Gew.-% und insbesondere 0,4 bis 0,8 Gew.-% Cocoamidopropylbetain.

Die erfindungsgemäßen Mund- und Zahnpflege- und -reinigungsmittel können weitere Inhaltsstoffe enthalten. Bevorzugt ist hierbei der Einsatz von sogenannten Feuchthaltemitteln, die bei Zahnpasten das Austrocknen verhindern. Bei sogenannten Flüssigzahncremes mit fließfähiger Rheologie dienen diese als Matrix und werden in höheren Mengen eingesetzt. Bei Mundwässern und Mundspülungen dienen diese Alkohole als Konsistenzregler und zusätzliche Süßungsmittel. Hier sind erfindungsgemäße Mund- und Zahnpflege- und -reinigungsmittel bevorzugt, die - bezogen auf ihr Gewicht - 0,5 bis 60 Gew.-%, vorzugsweise 0,75 bis 55 Gew.-%, besonders bevorzugt 1 bis 50 Gew.-% und insbesondere 2 bis 40 Gew.-% mindestens eines mehrwertigen Alkohols aus der Gruppe Sorbit und/oder Glycerin und/oder 1,2-Propylenglycol.-% oder deren Mischungen enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1) : (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Sorbit, so dass Mittel, die außer Sorbit keine anderen mehrwertigen Alkohole enthalten, besonders bevorzugt sind.

Die erfindungsgemäßen Mittel können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.
Mund- und Zahnpflege- und reinigungsmittel können z.B. auch Substanzen enthalten, die gegen Plaque und/oder Zahnstein wirksam sind.
Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P₂O₇, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.
Erfindungsgemäß bevorzugte Mund- und Zahnpflege- und -reinigungsmittel sind dadurch gekennzeichnet, dass sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.
Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Ganz besonders gut verträglich mit der erfindungsgemäßen Kombination ist Xanthan. Erfindungsgemäße Mittel, die Xanthan enthalten, sind außergewöhnlich lagerstabil und weisen eine angenehme Produkthaptik auf. Bevorzugte erfindungsgemäße Mund und Zahnpflege- und - reinigungsmittel sind daher dadurch gekennzeichnet, dass sie zusätzlich 0,1 bis 7,5 Gew.-%, vorzugsweise 0,25 bis 5 Gew.-%, weiter bevorzugt 0,5 bis 2,5 Gew.-% und insbesondere 0,6 bis 1,5 Gew.-% Xanthan enthalten.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose.
Weitere übliche Hilfs- und Zusatzstoffe für Zahnpasten und Mundwässer oder Mundspüllösungen sind
- Oberflächenaktive Stoffe, bevorzugt anionische, zwitterionische, amphotere, nichtionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldi- glycol
- Pigmente, wie z.B. Titandioxid
- Farbstoffe
- Puffersubstanzen, z.B. primäre, sekundäre oder tertiäre Alkaliphosphate oder Citronensäure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Erfindungsgemäße Mittel können als Zahnpasten oder Zahncremes formuliert werden. In Verfahren zur Vorbeugung vor und Behandlung von empfindlichen Zähnen wird eine erfindungsgemäße Zubereitung auf eine Zahnbürste aufgetragen und mit dieser die Zähne geputzt. Die erfindungsgemäßen Zusammensetzungen lassen sich auch als Mundspüllösungen oder Mundwässer formulieren. In Verfahren zur Vorbeugung vor und Behandlung von empfindlichen Zähnen wird eine erfindungsgemäße Zubereitung in Form einer Mundspüllösung in die Mundhöhle eingebracht und dort für einen Zeitraum von mindestens 10 Sekunden, vorzugsweise mindestens 20 Sekunden und insbesondere mindestens 45 Sekunden belassen.

Illustrative, nicht erfindungsgemäße Formulierungsbeispiele: Alle Angaben in Gew.-%

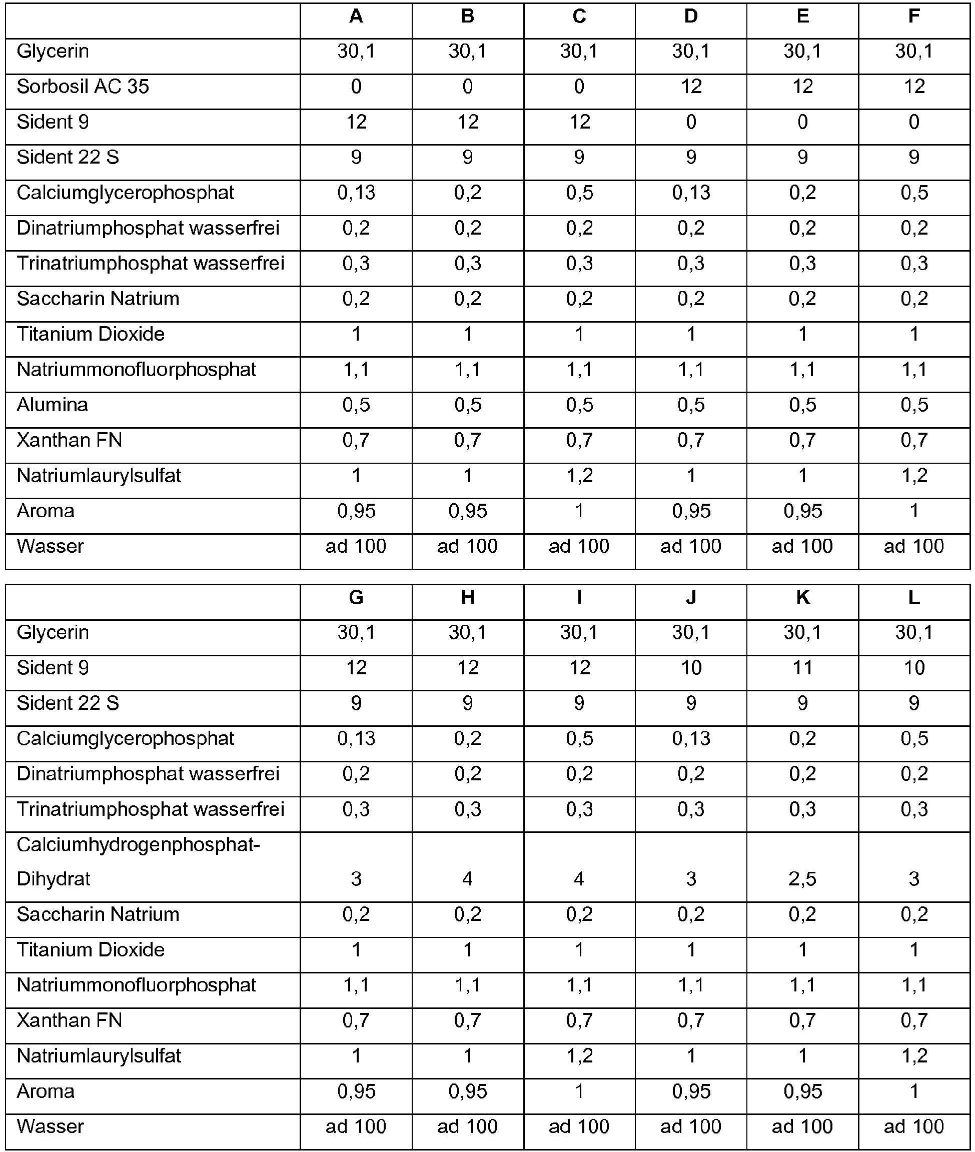

## Patentansprüche

1. Mund- und Zahnpflege- und -reinigungsmittel, enthaltend - bezogen auf sein Gewicht -
a) 1 bis 30 Gew.-% Fällungskieselsäure(n) mit einer spezifischen Oberfläche nach ISO 5794-1, Anhang D von ≤ 55 m²/g;
b) 0,1 bis 10 Gew.-% Natriummonofluorphosphat;
c) 0,001 bis 10,0 Gew.-% mindestens eines Calciumsalzes
wobei
das Gewichtsverhältnis von Fällungskieselsäuren mit einer spezifischen Oberfläche nach ISO 5794-1, Anhang D von ≤ 55 m²/g (Inhaltsstoff a)) zu ggf. vorhandenen Fällungskieselsäuren mit einer spezifischen Oberfläche nach ISO 5794-1, Anhang D von > 55 m²/g > 50:1 beträgt.

2. Mund- und Zahnpflege- und -reinigungsmittel nach Anspruch 1, **dadurch gekennzeichnet, dass** es 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 55 m²/g enthält.

3. Mund und Zahnpflege- und -reinigungsmittel nach einem der Ansprüche 1 oder 2, **dadurch gekennzeichnet, dass** sämtliche im Mittel enthaltenen Fällungskieselsäure(n) eine spezifische Oberfläche nach ISO 5794-1, Anhang D von ≤ 53 m²/g, vorzugsweise von ≤ 51 m²/g, weiter bevorzugt von ≤ 49 m²/g und insbesondere von ≤ 47 m²/g aufweisen.

4. Mund und Zahnpflege- und -reinigungsmittel nach einem der Ansprüche 1 bis 3, **dadurch gekennzeichnet, dass** es 0,01 bis 5 Gew.-%, vorzugsweise 0,1 bis 3 Gew.-%, weiter bevorzugt 0,2 bis 2 Gew.-% und insbesondere 0,5 bis 1,2 Gew.-% Natriummonofluorphosphat enthält.

5. Mund und Zahnpflege- und -reinigungsmittel nach einem der Ansprüche 1 bis 4, **dadurch gekennzeichnet, dass** es 0,05 bis 7,5 Gew.-%, vorzugsweise 0,1 bis 5 Gew.-%, weiter bevorzugt 0,15 bis 2,5 Gew.-% und insbesondere 0,2 bis 1,25 Gew.-% Calciumsalz(e) enthält.

6. Mund und Zahnpflege- und -reinigungsmittel nach einem der Ansprüche 1 bis 5, **dadurch gekennzeichnet, dass** es 0,01 bis 2,5 Gew.-%, vorzugsweise 0,05 bis 2,0 Gew.-%, besonders bevorzugt 0,1 bis 1,0 Gew.-%, weiter bevorzugt 0,11 bis 0,75 Gew.-% und insbesondere 0,12 bis 0,5 Gew.-% Calcium-Glycerophosphat enthält.

7. Mund und Zahnpflege- und -reinigungsmittel nach einem der Ansprüche 1 bis 6, **dadurch gekennzeichnet, dass** es 0,25 bis 7,5 Gew.-%, vorzugsweise 0,5 bis 7,0 Gew.-%, besonders bevorzugt 1,0 bis 6,0 Gew.-%, weiter bevorzugt 2,0 bis 5,0 Gew.-% und insbesondere 3,5 bis 4,5 Gew.-% Calciumhydrogenphosphat-Dihydrat enthält.

8. Mund und Zahnpflege- und -reinigungsmittel nach Anspruch 7, **dadurch gekennzeichnet, dass** es zusätzlich Magnesiumsulfat-Heptahydrat enthält und das Gewichtsverhältnis von Magnesiumsulfat-Heptahydrat zu Calciumhydrogenphosphat-Dihydrat 20:1 bis 1:1, vorzugsweise 15:1 bis 2:1, weiter bevorzugt 10:1 bis 5:2, besonders bevorzugt 4:1 bis 3:1 und insbesondere 18:7 bis 15:7 beträgt.

9. Mund und Zahnpflege- und -reinigungsmittel nach einem der Ansprüche 1 bis 8, **dadurch gekennzeichnet, dass** es zusätzlich 0,1 bis 7,5 Gew.-%, vorzugsweise 0,25 bis 5 Gew.-%, weiter bevorzugt 0,5 bis 2,5 Gew.-% und insbesondere 0,6 bis 1,5 Gew.-% Xanthan enthält.

10. Mund und Zahnpflege- und -reinigungsmittel nach einem der Ansprüche 1 bis 7, **dadurch gekennzeichnet, dass** es - bezogen auf sein Gewicht - weniger als 5 Gew.-%, vorzugsweise weniger als 4 Gew.-%, besonders bevorzugt weniger als 3 Gew.-% und insbesondere weniger als 2 Gew.-% Tensid(e) enthält.

## Claims

1. An oral and dental care and cleaning agent, containing, based on its weight,
a) 1 to 30 wt.% precipitated silica(s) having a specific surface area according to ISO 5794-1, Annex D, of ≤ 55 m²/g;
b) 0.1 to 10 wt.% sodium monofluorophosphate;
c) 0.001 to 10.0 wt.% of at least one calcium salt
wherein
the weight ratio of precipitated silicas having a specific surface area according to ISO 5794-1, Annex D, of ≤ 55 m²/g (ingredient a)) to optionally present precipitated silicas having a specific surface area according to ISO 5794-1, Annex D, of > 55 m²/g is > 50:1.

2. The oral and dental care and cleaning agent according to claim 1, **characterized in that** it contains 2.5 to 25 wt.%, preferably 5 to 20 wt.%, particularly preferably 7.5 to 17.5 wt.%, more preferably 8.0 to 15.0 wt.% and in particular from 10.0 to 14.0 wt.%, precipitated silica(s) having a specific surface area according to ISO 5794-1, Annex D, of ≤ 55 m²/g.

3. The oral and dental care and cleaning agent according to one of claims 1 or 2, **characterized in that** all the precipitated silica(s) contained in the agent has/have a specific surface area according to ISO 5794-1, Annex D, of ≤ 53 m²/g, preferably of ≤ 51 m²/g, more preferably of ≤ 49 m²/g and in particular of ≤ 47 m²/g.

4. The oral and dental care and cleaning agent according to one of claims 1 to 3, **characterized in that** it contains 0.01 to 5 wt.%, preferably 0.1 to 3 wt.%, more preferably 0.2 to 2 wt.% and in particular 0.5 to 1.2 wt.%, sodium monofluorophosphate.

5. The oral and dental care and cleaning agent according to one of claims 1 to 4, **characterized in that** it contains 0.05 to 7.5 wt.%, preferably 0.1 to 5 wt.%, more preferably 0.15 to 2.5 wt.% and in particular 0.2 to 1.25 wt.%, calcium salt(s).

6. The oral and dental care and cleaning agent according to one of claims 1 to 5, **characterized in that** it contains 0.01 to 2.5 wt.%, preferably 0.05 to 2.0 wt.%, particularly preferably 0.1 to 1.0 wt.%, more preferably 0.11 to 0.75 wt.% and in particular 0.12 to 0.5 wt.%, calcium glycerophosphate.

7. The oral and dental care and cleaning agent according to one of claims 1 to 6, **characterized in that** it contains 0.25 to 7.5 wt.%, preferably 0.5 to 7.0 wt.%, particularly preferably 1.0 to 6.0 wt.%, more preferably 2.0 to 5.0 wt.% and in particular 3.5 to 4.5 wt.%, calcium hydrogen phosphate dihydrate.

8. The oral and dental care and cleaning agent according to claim 7, **characterized in that** it also contains magnesium sulfate heptahydrate and the weight ratio of magnesium sulfate heptahydrate to calcium hydrogen phosphate dihydrate is 20:1 to 1:1, preferably 15:1 to 2:1, more preferably 10:1 to 5:2, particularly preferably 4:1 to 3:1 and in particular 18:7 to 15:7.

9. The oral and dental care and cleaning agent according to one of claims 1 to 8, **characterized in that** it also contains 0.1 to 7.5 wt.%, preferably 0.25 to 5 wt.%, more preferably 0.5 to 2.5 wt.% and in particular 0.6 to 1.5 wt.%, xanthan gum.

10. The oral and dental care and cleaning agent according to one of claims 1 to 7, **characterized in that** it contains, based on its weight, less than 5 wt.%, preferably less than 4 wt.%, particularly preferably less than 3 wt.% and in particular less than 2 wt.%, surfactant(s).

## Revendications

1. Produit de nettoyage et d'entretien de la bouche et des dents contenant - en fonction de son poids -
a) de 1 à 30 % en poids d'acide(s) silicique(s) précipité(s) ayant une surface spécifique, selon ISO 5794-1, annexe D, inférieure ou égale à 55 m²/g ;
b) de 0,1 à 10 % en poids de monofluorophosphate de sodium ;
c) de 0,001 à 10,0 % en poids d'au moins un sel de calcium
dans lequel
le rapport en poids entre des acides siliciques précipités ayant une surface spécifique, selon ISO 5794-1, annexe D, inférieure ou égale à 55 m²/g (composant a)) et des acides siliciques précipités éventuellement présents ayant une surface spécifique, selon ISO 5794-1, annexe D, supérieure à 55 m²/g, est de 50:1.

2. Produit de nettoyage et d'entretien de la bouche et des dents selon la revendication 1, **caractérisé en ce qu'**il contient de 2,5 à 25 % en poids, de préférence de 5 à 20 % en poids, de manière particulièrement préférée de 7,5 à 17,5 % en poids, de manière davantage préférée de 8,0 à 15,0 % en poids et en particulier de 10,0 à 14,0 % en poids d'acide(s) silicique(s) précipité(s) ayant une surface spécifique, selon ISO 5794-1, annexe D, inférieure ou égal à 55 m²/g.

3. Produit de nettoyage et d'entretien de la bouche et des dents selon l'une des revendications 1 ou 2, **caractérisé en ce que** la totalité de l'acide / des acides silicique(s) précipité(s) présent(s) dans le produit comporte(nt) une surface spécifique, selon ISO 5794-1, annexe D, inférieure ou égale à 53 m²/g, de préférence inférieure ou égale à 51 m²/g, de manière davantage préférée inférieure ou égale à 49 m²/g et en particulier inférieure ou égale à 47 m²/g.

4. Produit de nettoyage et d'entretien de la bouche et des dents selon l'une des revendications 1 à 3, **caractérisé en ce qu'**il contient de 0,01 à 5 % en poids, de préférence de 0,1 à 3 % en poids, de manière davantage préférée de 0,2 à 2 % en poids, et en particulier de 0,5 à 1,2 % en poids de monofluorophosphate de sodium.

5. Produit de nettoyage et d'entretien de la bouche et des dents selon l'une des revendications 1 à 4, **caractérisé en ce qu'**il contient de 0,05 à 7,5 % en poids, de préférence de 0,1 à 5 % en poids, de manière davantage préférée de 0,15 à 2,5 % en poids, et en particulier de 0,2 à 1,25 % en poids de sel(s) de calcium.

6. Produit de nettoyage et d'entretien de la bouche et des dents selon l'une des revendications 1 à 5, **caractérisé en ce qu'**il contient de 0,01 à 2,5 % en poids, de préférence de 0,05 à 2,0 % en poids, de manière particulièrement préférée de 0,1 à 1,0 % en poids, de manière davantage préférée de 0,11 à 0,75 % en poids, et en particulier de 0,12 à 0,5 % en poids de glycérophosphate de calcium.

7. Produit de nettoyage et d'entretien de la bouche et des dents selon l'une des revendications 1 à 6, **caractérisé en ce qu'**il contient de 0,25 à 7,5 % en poids, de préférence de 0,5 à 7,0 % en poids, de manière particulièrement préférée de 1,0 à 6,0 % en poids, de manière davantage préférée de 2,0 à 5,0 % en poids, et en particulier de 3,5 à 4,5 % en poids de dihydrate d'hydrogénophosphate de calcium.

8. Produit de nettoyage et d'entretien de la bouche et des dents selon la revendication 7, **caractérisé en ce qu'**il contient en outre du heptahydrate de sulfate de magnésium et le rapport pondéral entre l'heptahydrate de sulfate de magnésium et le dihydrate d'hydrogénophosphate de calcium est de 20:1 à 1:1, de préférence de 15:1 à 2:1, de manière davantage préférée de 10:1 à 5:2, de manière particulièrement préférée de 4:1 à 3:1, et en particulier de 18:7 à 15:7.

9. Produit de nettoyage et d'entretien de la bouche et des dents selon l'une des revendications 1 à 8, **caractérisé en ce qu'**il contient en outre de 0,1 à 7,5 % en poids, de préférence de 0,25 à 5 % en poids, de manière davantage préférée de 0,5 à 2,5 % en poids, et en particulier de 0,6 à 1,5 % en poids de gomme xanthane.

10. Produit de nettoyage et d'entretien de la bouche et des dents selon l'une des revendications 1 à 7, **caractérisé en ce qu'**il contient, par rapport à son poids, moins de 5 % en poids, de préférence moins de 4 % en poids, de manière particulièrement préférée moins de 3 % en poids, et en particulier moins de 2 % en poids de tensioactif(s).
